# EUROPÄISCHE PATENTSCHRIFT

(11) **EP 2 462 962 B1**
(45) Veröffentlichungstag und Bekanntmachung des Hinweises auf die Patenterteilung: **20.09.2017**
(21) Anmeldenummer: 11189695.7
(22) Anmeldetag: 18.11.2011
(51) Int. Cl.: A61L 31/02, A61L 31/08, A61L 31/14, A61L 31/16

(54) **IMPLANTAT MIT EINER DAS IMPLANTAT ZUMINDEST BEREICHSWEISE BEDECKENDEN, WIRKSTOFFHALTIGEN BESCHICHTUNG**
IMPLANT COMPRISING AN ACTIVE-AGENT-CONTAINING COATING COVERING THE IMPLANT AT LEAST IN SECTIONS
IMPLANT DOTÉ D'UN REVÊTEMENT COMPRENANT UN PRINCIPE ACTIF ET RECOUVRANT L'IMPLANT AU MOINS PARTIELLEMENT

(30) Priorität: 08.12.2010 US 420805 P
(43) Veröffentlichungstag der Anmeldung: 13.06.2012
(73) Patentinhaber: Biotronik AG, 8180 Bülach (CH)
(72) Erfinder: Gratz, Matthias, 91054 Erlangen (DE); Borck, Alexander, 91086 Aurachtal (DE); Rzany, Alexander, 90449 Nürnberg (DE); Schmiedl, Robert, 96114 Hirschaid (DE); Fringes, Matthias, 91522 Ansbach (DE); Harder, Claus, 91080 Uttenreuth (DE)
(74) Vertreter: Galander, Marcus

(56) Entgegenhaltungen:
- EP-A2- 2 070 558
- US-A1- 2006 155 370
- US-A1- 2007 244 548
- US-A1- 2009 024 200

## Beschreibung

Die Erfindung betrifft ein Implantat mit einer das Implantat zumindest bereichsweise bedeckenden, wirkstoffhaltigen Beschichtung.

Implantate haben in vielfältiger Ausführungsform Anwendung in der modernen Medizintechnik gefunden. Sie dienen beispielsweise der Unterstützung von Gefäßen, Hohlorganen und Gangsystemen (Endovaskuläre Implantate, z.B. Stents), zur Befestigung und temporären Fixierung von Gewebeimplantaten und Gewebstransplantationen, aber auch zu orthopädischen Zwecken, zum Beispiel als Nagel, Platte oder Schraube. Eine besonders häufig verwendete Form eines Implantats ist der Stent.

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die vorrangig zur Abdichtung des Aneurysmas dienen. Die Stützfunktion ist zusätzlich gegeben.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Alternativ besitzen Form-Gedächtnis-Materialien wie Nitinol die Fähigkeit zur Selbstexpansion, wenn eine Rückstellkraft wegfällt, die das Implantat auf einem kleinen Durchmesser hält. Die Rückstellkraft wird in der Regel durch einen Schutzschlauch auf das Material ausgeübt.

Das Implantat, insbesondere der Stent, besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßen Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatwerkstoffs ist weiterhin abhängig vom zeitlichen Ablauf der Reaktion des Biosystems, in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatwerkstoffs in verschiedener Weise. Entsprechend der Reaktion des Biosystems können die Implantatwerkstoffe in bioaktive, bioinerte und degradierbare/resorbierbare (hier als biokorrodierbar bezeichnete) Werkstoffe unterteilt werden.

Implantatwerkstoffe umfassen Polymere, metallische Werkstoffe und keramische Materialien (zum Beispiel als Beschichtung). Biokompatible Metalle und Metalllegierungen für Permanentimplantate beinhalten beispielsweise rostfreie Stähle (zum Beispiel 316L), Kobaltbasislegierungen (zum Beispiel CoCrMo-Gußlegierungen, CoCrMo-Schmiedelegierungen, CoCrWNi-Schmiedelegierungen und CoCrNiMo-Schmiedelegierungen), Reintitan und Titanlegierungen (z. B. cp Titan, TiA16V4 oder Ti-A16Nb7), Nickel-Titan-Legierungen (z.B. NiTiNol) und Goldlegierungen. Im Bereich biokorrodierbarer Stents wird der Einsatz von Magnesium oder Reineisen sowie biokorrodierbaren Basislegierungen der Elemente Magnesium, Eisen, Zink, Molybdän und Wolfram vorgeschlagen.

Bekannt ist, dass sich ein höheres Maß an Biokompatibilität erreichen lässt, wenn Implantatwerkstoffe mit Beschichtungen aus besonders gewebsverträglichen Materialien versehen werden. Diese Materialien sind zumeist organischer oder synthetisch-polymerer Natur und teils natürlichen Ursprungs. Weitere Strategien zur Vermeidung der Restenose konzentrieren sich darauf, die Proliferation durch Medikation, z. B. Behandlung mit Cytostatika, zu hemmen. Die Wirkstoffe können zum Beispiel auf der Implantatoberfläche in Form einer Wirkstoff-freisetzenden Beschichtung bereitgestellt werden.

Die Wirkstoffe werden entweder direkt als Beschichtung aufgetragen oder in eine Elutionsmatrix eingebettet. Insbesondere bei Implantaten aus biokorrodierbaren Materialien sollte auch die Elutionsmatrix in vivo abbaubar sein. Abbaubaren wie auch permanenten Polymeren, die bei wirkstoffeluierenden Implantaten zumeist als Elutionsmatrix verwendet werden, wird als Nachteil zugeschrieben, dass sie langfristig Entzündungsreaktionen am Implantationsort hervorrufen und dadurch das klinische Ergebnis beeinträchtigen. Daher sollte nach Möglichkeit auf eine polymerbasierte Elutionsmatrix verzichtet werden.

Implantate ohne polymerbasierte Elutionsmatrix sind aus dem Stand der Technik grundsätzlich bekannt. Dabei kann die - zumeist antiproliferativ wirkende - Substanz entweder in Reinform oder in einem Gemisch mit einem Exzipienten (Hilfsstoff als Träger) direkt auf die Oberfläche des Implantats aufgebracht werden, die wiederum glatt, aufgeraut, oder mit strukturellen Vertiefungen versehen sein kann, die als Reservoir dienen.

Solche Implantate haben jedoch den Nachteil, dass das Einwachsen in die Gefäßwand verzögert, langfristig nur teilweise oder überhaupt nicht stattfindet. Dadurch ergibt sich zum Beispiel langfristig das Risiko der Stent-Thrombose, der in der klinischen Praxis durch eine systemische Antikoagulationstherapie begegnet wird. Diese "Dual-Anti-Platelet-Therapie" (DAPT) hat zahlreiche klinische Nachteile, die man letztlich durch das verzögerte (oder ganz ausbleibende) Einwachsen konventioneller Implantate in Kauf nehmen muss.

US 2007/0244548 A1 bezieht sich auf eine medizinische Vorrichtung mit einer Oberfläche aus einer Schicht, welche ein Mono- oder Disaccharid und mindestens einen therapeutischen Wirkstoff enthält.

US 2009/0024200 A1 beschreibt einen Stent umfassend ein Gerüst und einen therapeutischen Wirkstoff eluierenden Bereich, wobei der Wirkstoff eluierende Bereich eine erste auf dem Stentgerüst angeordnete Zuckerschicht, mindestens eine darauf aufgebrachte wirkstoffhaltige Schicht, und eine zweite darauf angeordnete Zuckerschicht enthält.

US 2006/0155370 A1 bezieht sich auf einen Stent mit einer intermittierenden Beschichtung, das heißt mit einer Beschichtung mit einer Vielzahl von diskreten Beschichtungsabschnitten. Diese können verschiedene therapeutische Wirkstoffe und Polymere enthalten und können aus verschiedenen Lösungsmitteln hergestellt werden. Die Beschichtungsabschnitte können in Mustern aufgebracht werden.

EP 2 070 558 A2 beschreibt ein Implantat umfassend einen Implantatgrundkörper, einer Primerschicht, die ganz oder teilweise auf der Oberfläche des Implantates aufgebracht ist, einer Wirkstoffschicht bestehend aus ein oder mehreren Wirkstoffen, die ganz oder teilweise auf der Oberfläche der Primerschicht aufgebracht ist, und einer diffusionskontrollierenden Schicht, die ganz oder teilweise auf die Wirkstoffschicht und gegebenenfalls die Primerschicht aufgebracht ist und die Diffusion des oder der Wirkstoffe der Wirkstoffschicht kontrolliert.

Ein oder mehrere der zuvor angesprochenen Nachteile des Standes der Technik werden mit Hilfe des erfindungsgemäßen Implantats gelöst oder zumindest gemindert. Das Implantat weist eine das Implantat zumindest bereichsweise bedeckende, wirkstoffhaltige Beschichtung auf. Die Beschichtung besteht aus zumindest zwei Teilbereichen, von denen ein erster Teilbereich den zumindest einen Wirkstoff enthält und ein zweiter Teilbereich aus einem Hilfsstoff besteht.

Der Erfindung liegt die Erkenntnis zugrunde, dass auf eine polymere Elutionsmatrix verzichtet werden kann, wenn an Stelle dessen der Wirkstoff strukturiert aufgebracht wird, wobei die nicht mit dem Wirkstoff beschichteten Bereiche der Beschichtung von einem Hilfsstoff abgedeckt werden. Die von dem Hilfsstoff und von dem Wirkstoff bedeckten Bereiche der Implantatoberfläche bilden demnach eine geschlossene Fläche. Der Hilfsstoff ist so ausgelegt, dass er sich unmittelbar nach Implantation auflöst. Dadurch werden die strukturierten Wirkstoffbereiche freigelegt, wobei sich die Oberfläche dieser Bereiche hin zum umgebenden Medium stark vergrößert und damit auch eine anfängliche Elutionscharakteristik des Wirkstoffs verbessert werden kann. Der Hilfsstoff ist während der Implantation jedoch noch vorhanden, so dass die Struktur während des Verbringens an dem Implantationsort und während der ggf. notwendigen Fixation am Implantationsort vor Abrieb und/oder Auswaschung geschützt ist.

Eine Beschichtung im Sinne der Erfindung ist eine zumindest abschnittsweise Auftragung der Komponenten der Beschichtung auf den Grundkörper des Implantats. Vorzugsweise ist im Falle eines Stents die Beschichtung nur abluminal auf dem Implantat aufgetragen. Eine Schichtdicke liegt vorzugsweise im Bereich von 1 nm bis 100 µm, besonders bevorzugt 300 nm bis 15 µm Die erfindungsgemäße Beschichtung kann direkt auf die Implantatoberfläche aufgetragen werden oder es können weitere Zwischenschichten vorgesehen werden; so kann gegebenenfalls der Grundkörper des Implantats eine anorganische Grundschicht (z.B. Sol-Gel-Beschichtungen) aufweisen, die die Haftung der erfindungsgemäßen Beschichtung verbessert. Auf die erfindungsgemäße Beschichtung können weitere Schichten aufgetragen werden, um zum Beispiel die Einführung des Implantats in den Körper zu erleichtern. Verfahren zur Beschichtung von Implantaten sind dem Fachmann bekannt (z.B. Aufpipettieren, Aufrollen). Zur Herstellung der erfindungsgemäßen Beschichtung kann zunächst ganzflächig der Hilfsstoff aufgetragen werden und dann diese Beschichtung im Sinne der Erfindung strukturiert werden. Die Bereiche, der Beschichtung, in denen der Hilfsstoff durch die Strukturierung entfernt wurde, werden dann mit dem Wirkstoff aufgefüllt. Das Strukturieren kann zum Beispiel durch Prägen, Strahlen oder Laser-/Temperaturpressen erfolgen.

Gemäß einer ersten, bevorzugten Ausführungsform sind die einzelnen Teilbereiche so ausgestaltet, dass sich ein Muster aus sich abwechselnden Streifen der ersten und zweiten Teilbereiche ergibt. Insbesondere liegt eine Breite der Streifen, die den ersten Teilbereich der Beschichtung bilden, im Bereich von 1 bis 30 µm Das Muster kann insbesondere regelmäßig ausgebildet sein. Die genannte Abmessung und Mustergestaltung hat sich als besonders effektiv herausgestellt, um das Einwachsverhalten eines Stents in die Gefäßwand zu beschleunigen.

Gemäß einer zweiten, bevorzugten Ausführungsform sind die einzelnen Teilbereiche so ausgestaltet, dass sich ein Muster aus vom zweiten Teilbereich umschlossenen Inseln aus dem ersten Teilbereich ergibt. Insbesondere liegt eine Breite oder ein Durchmesser der Inseln, die den ersten Teilbereich der Beschichtung bilden, im Bereich von 1 bis 30 µm. Das Muster kann insbesondere regelmäßig ausgebildet sein. Die genannte Abmessung und Mustergestaltung hat sich als besonders effektiv herausgestellt, um das Einwachsverhalten eines Stents in die Gefäßwand zu beschleunigen.

Vorzugsweise besteht ein Grundkörper des Implantats aus einem biokorrodierbaren Werkstoff, insbesondere aus einer biokorrodierbaren Magnesiumlegierung.

Als biokorrodierbar im Sinne der Erfindung werden Werkstoffe bezeichnet, bei denen in physiologischer Umgebung ein Abbau/Umbau stattfindet, so dass der aus dem Werkstoff bestehende Teil des Implantates danach ganz oder zumindest überwiegend nicht mehr vorhanden ist.

Unter Magnesiumlegierung wird vorliegend ein metallisches Gefüge verstanden, dessen Hauptkomponente Magnesium ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Die Legierung ist so in ihrer Zusammensetzung zu wählen, dass sie biokorrodierbar ist. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37 °C und pH 7,38 gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Implantate im Sinne der Erfindung sind über ein chirurgisches Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes und Ankerelemente für Elektroden, insbesondere von Schrittmachern oder Defibrillatoren. Das Implantat besteht ganz oder in Teilen aus dem biokorrodierbaren Werkstoff. Vorzugsweise ist das Implantat ein Stent.

Der erste Teilbereich der Beschichtung enthält einen oder mehrere Wirkstoffe, die nach der Implantation freigesetzt werden. Unter Wirkstoff im erfindungsgemäßen Sinne wird ein Arzneistoff mit pharmazeutischer Wirkung verstanden, der im menschlichen oder tierischen Körper zur Heilung, Linderung, Verhütung oder Erkennung von Krankheiten dient. Wirkstoffe umfassen insbesondere Paclitaxel, Sirolimus und dessen Derivate. Insbesondere sind Wirkstoffe vorteilhaft, die auf mTOR wirken, sowie RAS-Inhibitoren, insbesondere solche, die die RAS-Adhäsion verhindern.

Der Hilfsstoff ist vorzugsweise in vivo innerhalb von weniger als 10 h, insbesondere 2 h, vollständig lösbar. Der Hilfsstoff kann zu 80 Gew.% oder mehr aus einem Zucker oder Zuckerderivat bestehen. Beispielsweise können Gemische aus 6-O-α-D-Glucopyranosyl-D-sorbitol (1,6-GPS) und 6-O-α-D-Glucopyranosyl-D-mannitol dihydrat (1,1-GPM) Verwendung finden. Ebenso auch Isomalt, Milchzucker, Cellulosepulver, Mannitol, Calciumdiphosphat oder Sorbitol.

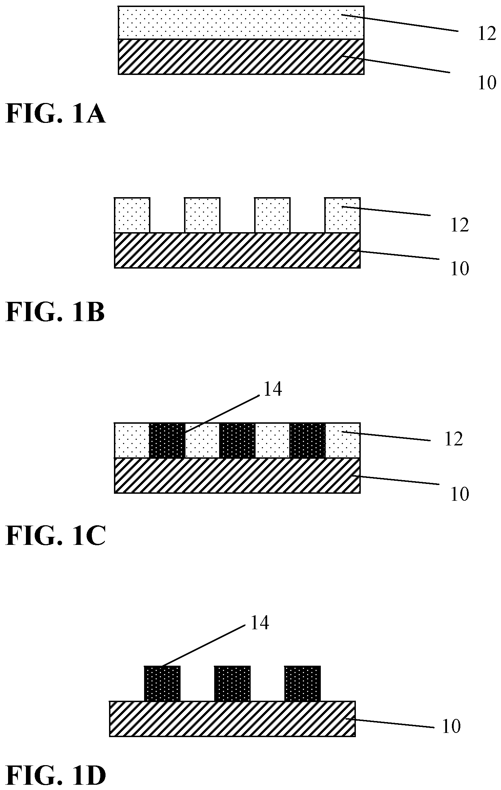

Die Erfindung wird nachfolgend anhand eines Ausführungsbeispiels und einer dazugehörigen Figur näher erläutert. Die einzige Figur zeigt schematisch einen Herstellungsweg für die erfindungsgemäße Beschichtung.

Die einzige Figur zeigt stark schematisiert in den Teilschritten A bis C eine Möglichkeit der Herstellung der erfindungsgemäßen Beschichtung und in der Teildarstellung D den Zustand nach erfolgter Implantation. Der Grundkörper des Implantats 10 wird im Teilschritt A zunächst mit dem Hilfsstoff 12 beschichtet, so dass eine geschlossene Oberfläche entsteht. In diese Oberfläche werden im Teilschritt B, z. B. durch Prägen, Rillen eingebracht, die wiederum im Teilschritt C mit dem Wirkstoff befüllt werden, so dass sich wiederum eine insgesamt geschlossene Beschichtung ergibt. Der Hilfsstoff 12 ist dabei so ausgelegt, dass er sich rasch nach Implantation auflöst, so dass stark schematisiert sich kurz nach der Implantation eine Struktur wie im Teilschritt D dargestellt ergibt.

### Ausführungsbeispiel

Ein Stent wird im Sprühverfahren mit einer Beschichtung aus dem Hilfsstoff versehen. Der Hilfsstoff ist eine 1:1 Mischung von 6-O-α-D-Glucopyranosyl-D-sorbitol (1,6-GPS) und 6-O-α-D-Glucopyranosyl-D-mannitol dihydrat (1,1-GPM), welche für das Sprühverfahren in Wasser gelöst wird. Dieser Hilfsstoff ist für galenische Anwendungen unter den Namen galenIQ® oder Palatinit erhältlich. Er lässt sich gut unter Druck strukturieren und löst sich, anders als Maisstärke, ohne Komplikationen im Blutstrom auf. Die Schichtdicke beträgt hierbei 200 nm bis 5 µm, bevorzugt 1 bis 3 µm. Durch Prägen werden in die Beschichtung aus dem Hilfsstoff Rillen mit einer Breite von etwa 1 - 30 µm eingebracht. Anschließend werden die Rillen mit dem Wirkstoff befüllt. Hierfür wird Paclitaxel oder Rapamycin in Verbindung mit einem Exzipienten aus der Gruppe Iopromide (Sequent Please), FreePac (Invatec), Butyryl-n-trihexyl-citrat (BTHC) oder Triethylcitrat verwendet. Nach Implantation löst sich der Hilfsstoff rasch auf und die Wirkstoffschicht bleibt übrig.

## Patentansprüche

1. Implantat mit einer das Implantat zumindest bereichsweise bedeckenden, wirkstoffhaltigen Beschichtung, wobei die Beschichtung aus zumindest zwei Teilbereichen besteht, von denen ein erster Teilbereich den zumindest einen Wirkstoff enthält und ein zweiter Teilbereich aus einem Hilfsstoff besteht, wobei der erste Teilbereich strukturiert vorliegt, und die vom ersten Teilbereich nicht beschichtete Oberfläche durch den zweiten Teilbereich so abgedeckt wird, dass eine geschlossene Fläche gebildet wird,
**dadurch gekennzeichnet, dass**
sich der Hilfsstoff unmittelbar nach der Implantation auflöst.

2. Implantat nach Anspruch 1, bei dem die einzelnen Teilbereiche so ausgestaltet sind, dass sich ein Muster aus sich abwechselnden Streifen der ersten und zweiten Teilbereiche ergibt.

3. Implantat nach Anspruch 2, bei dem eine Breite der Streifen, die den ersten Teilbereich der Beschichtung bilden, im Bereich von 1 bis 30 µm liegt.

4. Implantat nach Anspruch 1, bei dem die einzelnen Teilbereiche so ausgestaltet sind, dass sich ein Muster aus vom zweiten Teilbereich umschlossenen Inseln aus dem ersten Teilbereich ergibt.

5. Implantat nach Anspruch 4, bei dem eine Breite oder ein Durchmesser der Inseln, die den ersten Teilbereich der Beschichtung bilden, im Bereich von 1 bis 30 µm liegt.

6. Implantat nach Anspruch 2 oder 4, bei dem das Muster regelmäßig ausgebildet ist.

7. Implantat nach Anspruch 1, bei dem ein Grundkörper des Implantats aus einem biokorrodierbaren Werkstoff besteht.

8. Implantat nach Anspruch 7, bei dem der Grundkörper aus einer biokorrodierbaren Magnesiumlegierung besteht.

9. Implantat nach Anspruch 1, bei dem der Wirkstoff Paclitaxel, Sirolimus, ein Derivat von Sirolimus oder ein oder mehrere RAS-Inhibitoren ist.

10. Implantat nach Anspruch 1, bei dem der Hilfsstoff zu 80 Gew.% oder mehr aus einem Zucker oder Zuckerderivat besteht.

11. Implantat nach Anspruch 1, bei dem das Implantat ein Stent ist.

## Claims

1. An implant comprising an active-agent-containing coating covering the implant at least in sections, wherein the coating is composed of at least two subsections, of which a first subsection contains the at least one active agent, and a second subsection contains an auxiliary agent, wherein the first subsection is present in structured form and the surface not coated by the first subsection is covered by the second subsection such that a closed surface is formed,
**characterised in that**
the auxiliary agent dissolves immediately after implantation.

2. The implant according to claim 1, wherein the individual subsections are designed such that the result is a pattern of alternating strips of the first and the second subsections.

3. The implant according to claim 2, wherein a width of the strips that form the first subsection of the coating is in the range of 1 to 30 µm.

4. The implant according to claim 1, wherein the individual subsections are designed such that the result is a pattern of islands of the first subsection enclosed by the second subsection.

5. The implant according to claim 4, wherein a width or a diameter of the islands that form the first subsection of the coating is in the range of 1 to 30 µm.

6. The implant according to claim 2 or 4, wherein the pattern repeats.

7. The implant according to claim 1, wherein a main body of the implant is composed of a biocorrodible material.

8. The implant according to claim 7, wherein the main body is composed of a biocorrodible magnesium alloy.

9. The implant according to claim 1, wherein the active agent is selected from paclitaxel, sirolimus, a derivative of sirolimus, and one or more RAS inhibitors.

10. The implant according to claim 1, wherein the auxiliary agent comprises 80% or more by weight of a sugar or a sugar derivative.

11. The implant according to claim 1, wherein the implant is a stent.

## Revendications

1. Implant doté d'un revêtement contenant un principe actif recouvrant l'implant au moins partiellement, où le revêtement est constitué d'au moins deux régions partielles, parmi lesquelles une première région partielle contient l'au moins un principe actif et la seconde région partielle est constituée d'une substance auxiliaire, où la première région partielle apparaît structurée et la surface non revêtue de la première région partielle est recouverte par la deuxième région partielle de telle manière qu'une surface fermée est formée,
**caractérisé en ce que**
la substance auxiliaire se dissout immédiatement après l'implantation.

2. Implant selon la revendication 1, chez lequel les régions partielles individuelles sont conçues de telle manière qu'il se forme un motif constitué de bandes alternatives des première et seconde régions partielles.

3. Implant selon la revendication 2, chez lequel une largeur des bandes qui forment la première région partielle se situe dans la plage de 1 à 30 µm.

4. Implant selon la revendication 1, chez lequel les régions partielles individuelles sont conçues de telle manière qu'il se forme un motif à partir d'ilots constitués de la première région partielle entourés de la seconde région partielle.

5. Implant selon la revendication 4, chez lequel une largeur ou un diamètre des îlots qui forment la première région partielle du revêtement se situe dans la plage de 1 à 30 µm.

6. Implant selon la revendication 2 ou 4, chez lequel le motif est conçu de manière régulière.

7. Implant selon la revendication 1, chez lequel un corps de base de l'implant est constitué d'un principe actif biocorrodable.

8. Implant selon la revendication 7, chez lequel le corps de base est constitué d'un alliage de magnésium bicorrodable.

9. Implant selon la revendication 1, chez lequel le principe actif est du paclitaxel, du sirolimus, un dérivé de sirolimus ou un ou plusieurs inhibiteurs de RAS.

10. Implant selon la revendication 1, chez lequel la substance auxiliaire est constituée à 80 % en poids ou plus d'un sucre ou d'un dérivé de sucre.

11. Implant selon la revendication 1, chez lequel l'implant est un stent.
